# EUROPEAN PATENT APPLICATION

(11) **EP 0 622 082 A1**
(43) Date of publication of application: **02.11.1994**
(21) Application number: 93916235.0
(22) Date of filing: 28.07.1993
(51) Int. Cl.: A61K 39/395

(54) **IMMUNOCOMPLEX**

(30) Priority: 28.07.1992 JP 201545/92
(71) Applicant: TORAY INDUSTRIES, INC., Tokyo 103 (JP)
(72) Inventor: UENISHI, Noriaki, Kamakura-shi, Kanagawa 248 (JP); YAMAZAKI, Shojiro, Kamakura-shi, Kanagawa 248 (JP); KAJITA, Akemi, Fujisawa-shi, Kanagawa 251 (JP); NARUMI, Hideki, Kamakura-shi, Kanagawa 248 (JP); SONE, Saburo, Yokohama-shi, Kanagawa 244 (JP)
(74) Representative: Kador & Partner
(86) International application number: JP9301061
(87) International publication number: WO9402174

(57) **Abstract**

An immunocomplex which does not cleave *in vivo*, is stable, and retains a potent drug effect. The complex comprises an antibody or an antigen-binding fragment thereof and an abrin A chain, each of which is bound to a cross-linking agent which does not cleave *in vivo* through a thio ether bond.

## Description

### TECHNICAL FIELD

The present invention relates to an immunoconjugate effective for treating cancers; heart diseases and circulatory diseases; immune diseases and allergies; infectious diseases and the like.

### Prior Art

In addition to in vitro diagnostics, the use of monoclonal antibodies as in vivo diagnostics and in vivo therapeutic agents are now intensively studied. Thus, development of pharmaceuticals employing monoclonal antibodies are now intensively proceeded in the fields of therapies of tumors; heart and circulatory diseases; immune diseases and allergies; infectious diseases and the like. Particularly, diagnostics and therapeutic agents using monoclonal antibodies which specifically bind to tumor cells are now especially intensively studied and developed.

Therapeutic methods for treating cancers employing monoclonal antibodies include the methods in which an antibody alone is administered so as to gather macrophages and lymphocytes having receptors for Fc region of antibodies around the tumor region, thereby attacking the cancer; and the target therapeutic methods in which an immunoconjugate is used, that comprises a monoclonal antibody to which a toxic substance such as a cytotoxic protein including ribosome-inactivating proteins (hereinafter referred to as "RIP" for short) and some types of enzymes; a radioisotope (RI); an anti-cancer agent or the like is bound.

Among the latter target therapeutic methods, immunotoxin conjugates are especially intensively studied, and therapy of cancers by using conjugates having a tumor-specific monoclonal antibody and ricin A chain which is a plant toxin or a Pseudomonas exotoxin is now studied. Although it is most important that the immunotoxin conjugates have a low toxicity to the body and yet have an effective pharmacological effect, the stability of the administered drug in the blood is also important. For example, antibody-ricin A chain conjugate is mainly prepared by cross-linking the antibody and ricin A chain via N-succinimidyl 3-(2-pyridylthio)propionate (SPDP), and it is said that the disulfide bond (S-S bond) formed by the linker SPDP is indispensable for exhibiting strong pharmacological effect. However, the S-S bond has a drawback that it is attacked by another thiol compound in the blood and is easily cleaved. In order to overcome this problem, Thorpe et al developed a new linker 4-succinimidyloxycarbonyl-α-methyl-α-(2-pyridyldithio)toluene (SMPT) in which the S-S bond is sterically hindered by methyl group in the side chain. However, as long as the S-S bond exists, the cleavage of the S-S bond cannot be completely avoided. On the other hand, although it is tried to prepare immunotoxin conjugates employing a cross-linking agent forming thioether bond, such as succinimidyl-4-(N-maleimidomethyl)cyclohexane-1-carboxylate (SMCC) or N-(ε-maleimidocaproyloxy)succinimide (EMCS), effective pharmacological effect has not been obtained. This is one of the grounds that the S-S bond is said to be indispensable. Non-specific side effects caused by the liberated toxin, which do not relate to the specificity of the antibody, have been reported, so that there is a concern about the safety of the immunotoxin conjugates. Thus, an immunoconjugate which is not cleaved in the body and is stable, which retains a strong pharmacological effect is strongly demanded.

### DISCLOSURE OF THE INVENTION

Accordingly, an object of the present invention is to provide an immunoconjugate which is not cleaved and is stable in the body, and which retains a strong pharmacological effect.

That is, the present invention provides an immunoconjugate comprising an antibody or an antigen-binding fragment thereof and abrin A chain, said antibody or antigen-binding fragment thereof and said abrin A chain being bonded to a cross-linking agent via thioether bonds, respectively, which cross-linking agent is not cleaved in the body.

Since the immunoconjugate according to the present invention does not have a structure such as disulfide bond which is easily cleaved by being attacked by a thiol compound, or a peptide bond which is cleaved by an enzyme, the immunoconjugate is not decomposed when administered to human and during circulating in the blood and retains the stable structure. Therefore, the immunoconjugate according to the present invention reaches the target antigen without being decomposed, so that it exhibits high pharmacological effect.

### BRIEF DESCRIPTION OF THE DRAWINGS

Fig. 1 shows the change in the levels of two types of immunotoxin conjugates in the blood of mice with time after administering the immunotoxin conjugates, the immunotoxin conjugate level in the blood being expressed in terms of the ratio of the immunotoxin conjugate level in the blood to the administered dose.

Fig. 2 shows the results of the test by which the growth inhibition of tumor cells (QG56) by the two types of immunotoxin conjugates were examined.

Fig. 3 shows the change in the tumor weights (calculated from the diameters of the tumors) of tumor-bearing nude mice (7 mice per group) to which two types of immunotoxin conjugate were administered.

Fig. 4 shows the change in the tumor weights (calculated from the diameters of the tumors) of tumor-bearing nude mice (7 mice per group) to which an immunotoxin conjugate (Fab'-S-RA) was administered.

### BEST MODE FOR CARRYING OUT THE INVENTION

The antibody or the antigen-binding fragment thereof (A-SH) in the immunoconjugate according to the present invention has at least one thiol group in one molecule. If there is no thiol group in the molecule, the molecule is used after introducing one or more thiol groups in the molecule. In cases where the molecule has not less than two thiol groups, one thiol group may be retained and other cysteine residue(s) may be exchanged with another amino acid residue by a recombinant DNA technique. In cases where the molecule has a disulfide bond, the molecule may be used after reducing the disulfide bond to thiol groups. The methods for introducing thiol groups include a method in which SH group(s) is(are) chemically introduced, and a method in which cysteine (Cys) residue is introduced. A method in which Cys residue, especially free Cys residue is introduced is preferred. The Cys residue may be introduced chemically or by using recombinant DNA technique. These methods are known and described in, for example, S. Kanaya et al., J. Biol. Chem., 267, 8492 (1992). The antibody or the antibody fragment can be bound to the cross-linking agent hereinbelow described via thioether bond utilizing the SH group.

The antibody or the antibody fragment in the immunoconjugate according to the present invention may be various immunoglobulins such as IgG; and fragments thereof having specificity to the antigen, such as Fab', Fab and Fv. Among these, Fab' is preferred since the immunotoxin conjugate containing this fragment retains strong effect. Further, the antibody may be a human antibody obtained by genetic recombination technique, or a chimeric antibody in which the amino acid sequence of the constant region of an animal antibody is replaced with the amino acid sequence of the constant region of human antibody. It is preferred that these antibodies have at least one free Cys residue in the molecule.

In cases where the immunoconjugate according to the present invention is used as a diagnostic or a therapeutic agent for cancers, the antibody or the fragment thereof in the immunoconjugate preferably specifically binds to tumor cells, and the antibody is preferably a monoclonal antibody. The monoclonal antibody employed in the present invention may be any monoclonal antibody which specifically binds to a specific cell. By using an immunotoxin conjugate containing such a monoclonal antibody, the specific cell can be killed. It is preferred to employ a monoclonal antibody which is quickly internalized into the cell after the monoclonal antibody binds to the specific cell. A monoclonal antibody with which not less than 50%, more preferably not less than 60% of the monoclonal antibody is internalized into the cell is preferred.

As a monoclonal antibody which specifically binds to a tumor cell, it is preferred that not less than 80% of the monoclonal antibody is internalized into the tumor cell within 2 hours after the monoclonal antibody binds to the tumor cell. Further, a monoclonal antibody with which the cytotoxin component conjugated to the antibody effectively functions is preferred. As the monoclonal antibody, for example, monoclonal antibodies specific to glycoproteins on the surface of human hepatocarcinoma cells, such as AF20 and XF8 may be employed. AF20 (ATCC HB 9687) and XF8 (ATCC HB 9686) are monoclonal antibodies [H. Takahashi et al., Hepatology 9, 625 (1989)] which are specific to surface antigens on human hepatocarcinoma cell line FOCUS. It has been confirmed that these monoclonal antibodies specifically bind to human colorectal tumor cells, human lung tumor cells, human pancreatic tumor cells and blood tumor cells, in addition to human hepatic tumor cells.

The toxic substance contained in the immunoconjugate according to the present invention is A chain of abrin. Abrin A chain is obtained from abrin which is extracted and purified from seeds of Abrus precatorius. Abrin is a plant toxin which exhibits strong toxicity, and consists of two types of proteins, that is, abrin A chain and abrin B chain, that are covalently bonded via one disulfide bond. Abrin A chain (MW 30,000) has an activity to inactivate ribosomes by specifically hydrolyzing the N-glycoside bond between adenine and ribose of the 4324th nucleotide (A4324) of 28S rRNA in the 60S subunit of eukaryotic ribosomes. Abrin A chain does not have a sugar chain and its isoelectric point is 4.6 that is considerably lower than those of other RIPs, so that accumulation of abrin A chain in the liver can be avoided, which is preferred. A preferred example for purifying abrin A chain is detailed in the examples described below. Since abrin A chain has a free Cys residue, it can be bound to the cross-linking agent hereinbelow described via thioether bond utilizing the thioalcohol group in the Cys residue.

In the immunoconjugate according to the present invention, the above-described antibody or the fragment thereof and abrin A chain are bound to a cross-linking agent via thioether bond, respectively. This cross-linking agent has a stable structure which is not cleaved in the body. That is, the cross-linking agent does not have a structure which can be cleaved in the body, such as disulfide bond that may easily be cleaved by being attacked by a thiol compound, or peptide bond that may be cleaved by an enzyme.

Preferred examples of such a cross-linking agent include those represented by the following formula (II): 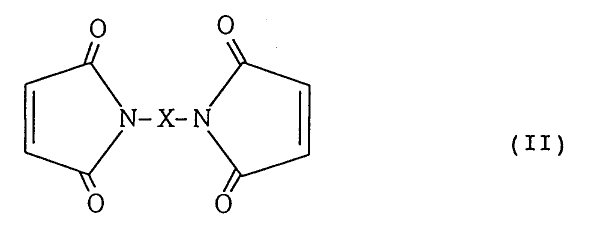
(wherein X represents a structure which is not cleaved in the body).

The compounds represented by the formula (II) have maleimide groups at their both ends, which react with SH group. The spacer moiety (X) linking the two maleimide groups has a stable non-cleaved structure, which is not cleaved by enzymes, oxidizing agents or reducing agents in human body. Examples of the spacer moiety include alkyl chains (CₙH₂ₙ₊₁, wherein n means an integer of 1 - 20), ethers (CₙH₂ₙ₊₁-O-CₘH₂ₘ₊₁, wherein n and m mean integers of 1 - 20), and water-soluble polymers. In addition, sulfur-containing compounds (e.g., bisalkylsulfones and dialkylthioethers) and nitrogen-containing compounds (e.g., alkylamines, azoalkyls and azobenzene) having the same size as the structures mentioned above may also be employed. A specific example of the cross-linking agent is bismaleimide methyl ether (B-MME). The spacer moiety (X) of this linker is dimethyl ether. In addition to BMME, dimaleimide linkers having various ether structures may be employed.

It is more preferred that the spacer moiety (X) of the linker have a high water-solubility so that the compound (II) has a sufficient water solubility. Examples of the cross-linking agent having such a water-soluble structure of X include dimaleimide linkers containing a polyethylene glycol, -(CH₂CH₂O)ₙ- (wherein n represents an integer of 1 - 200, more preferably an integer of 4 - 100) as the spacer moiety (X). In addition, the spacer moiety (X) may be a polyvinyl alcohol having a polymerization degree of 1 - 200, preferably 4 - 100, polyvinylpyrrolidone (PVP), a polysaccharide sugar chain such as dextran, cellulose, agarose or the like. It is also preferred that X has a symmetrical structure. For example, a cross-linking agent having isopropylalcohol as the spacer moiety (X) is preferred because it not only has a symmetrical structure of the molecule but also has a high water solubility.

The compounds (II) may be produced according to the method by Tawney et al [J. Org. Chem., 26, 15-21 (1961)] or modifications thereof.

The immunoconjugate according to the present invention may be obtained by, for example, the following method: That is, firstly, the antibody or the fragment thereof (A-SH) is reacted with the compound represented by the above-described formula (II) to obtain a compound represented by the formula (III): 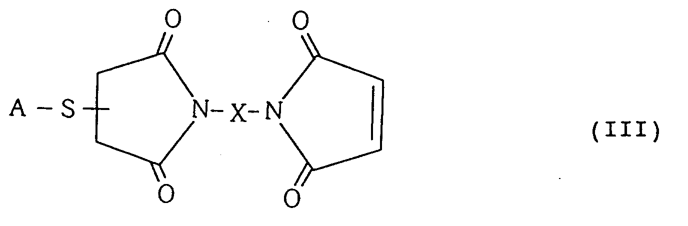
[wherein A represents a residue of antibody or the fragment thereof (A-SH), and X represents the same meanings as described above].
This reaction may be carried out by, for example, reacting the compound represented by the formula (II) with the antibody or the antibody fragment (A-SH) in a solvent such as 0.1 M phosphate buffer (pH 6.2) at a molar ratio of about 10:1 - 100:1 at a temperature of about 15 - 35^{o}C for 0.5 - 2 hours.

Then the obtained reaction product and abrin A chain (since abrin A chain has a free thioalcohol group, it can be expressed as B-SH) to obtain the immunoconjugate represented by the formula (I) according to the present invention. This reaction may be carried out by, for example, reacting the above-mentioned reaction product with abrin A chain (B-SH) in a solvent such as 0.1 M phosphate buffer (pH 6.2) at a molar ratio of about 1:1 - 1:5 at a temperature of about 15 - 35^{o}C for 3 - 24 hours.

Alternatively, the immunoconjugate represented by the formula (I) according to the present invention may be produced by firstly reacting the compound represented by the formula (II) with abrin A chain and reacting the obtained reaction product with the antibody or the fragment thereof. In this case, the conditions of each reaction may be the same as those described above.

### Examples

The present invention will now be described in more detail by way of examples thereof. It should be noted that the examples are presented for the illustration purpose only and should not be interpreted in any restrictive way.

### Example 1 Preparation of Immunoconjugate

### A. Antibody

As the antibody which specifically binds to a tumor cell, monoclonal antibody AF20 specific to the surface antigen on the human hepatocarcinoma cell line FOCUS, which was prepared by the method described in H. Takahashi et al., Hepatology 9, 625 (1989)] was employed.

As for the behavior of this monoclonal antibody after binding to human lung squamous cell carcinoma QG56, especially as for the uptake of this monoclonal antibody into the cells, it has been confirmed that the monoclonal antibody is internalized into the cells within 60 - 120 minutes and the antibody bound to the cell surface is quickly decreased, as described in Japanese Laid-open Patent Application (Kokai) No. 2-173516.

Fab' fragment of this monoclonal antibody was prepared as follows: That is, bromelain (commercially available from SIGMA) was incubated in 0.05 M Tris buffer (pH 7.0) containing 0.1 M cysteine and 0.002 M EDTA at 37^{o}C for 1 hour and cysteine was removed by gel permeation chromatography on SEPHADEX G-25 (commercially available from PHARMACIA). AF20 antibody was treated with the resulting bromelain at 37^{o}C for 2 hours. Thereafter, bromelain and decomposed Fc region were removed from the reaction product by SEPHACRYL S-200HR (commercially available from PHARMACIA). From the resultant, non-reacted IgG was further removed by chromatography on Q-SEPHAROSE (commercially available from PHARMACIA)/0.05 M Tris buffer (pH 8.0) or on PROTEIN A - SEPHAROSE CL-4B (commercially available from PHARMACIA)/0.1 M phosphate buffer (pH 8.0) to obtain F(ab')₂ fragment. The obtained F(ab')₂ fragment was treated with 1 mM 2-mercaptoethylamine at room temperature for 1 hour to reduce the disulfide bond at the hinge region of F(ab')₂ to obtain Fab' fragment. The Fab' fragment of the antibody has two free Cys residues.

### B. Abrin A Chain

Abrin A chain (AA) was extracted and purified as follows: That is, seeds of Abrus precatorius were milled and the resultant was defatted with petroleum ether, immediately followed by extraction with water. In the extraction, the pH was adjusted to 4.5 by acetic acid. The resultant was centrifuged and the supernatant was recovered. The supernatant was subjected to ammonium sulfate precipitation (40 - 70%), acid-treated SEPHAROSE 4B (commercially available from PHARMACIA), and then to affinity chromatography on DEAE cellulose to purify abrin. The obtained abrin was reduced in Tris buffer (pH 7.7) containing 5% mercaptoethanol at room temperature for 3 hours to overnight and then the pH was adjusted to 8.5. The resultant was then loaded on DEAE cellulose column and the column was well washed. The abrin A chain was eluted with 0 - 0.2 M NaCl buffer (pH 8.5) and the obtained fraction containing abrin A chain was passed through asialofetuin column, followed by gel permeation chromatography to obtain purified abrin A chain. Analysis by SDS-PAGE and subsequent staining with silver revealed that the obtained abrin A chain was not contaminated with abrin B chain. The abrin A chain has one free Cys residue.

### C. Preparation of Immunotoxin Conjugate

The following two types of conjugates containing AF20(Fab') and abrin A chain were prepared:
(1) A conjugate represented by the formula (IV) in which Fab' and AA are cross-linked via S-S bond.

   A-S-S-B (IV)

   (This conjugate is hereinafter referred to as "Fab'-SS-AA").
(2) A conjugate represented by the formula (V) in which Fab' and AA are cross-linked via thioether bond.

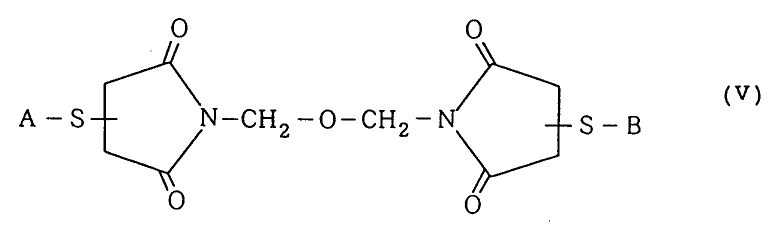

(This conjugate is hereinafter referred to as "Fab'-S-AA" for short).

These conjugates were prepared as follows:
Fab'-SS-AA was prepared by treating Fab' with 2 mM DTNB (5,5'-dinitro-bis-(2-nitrobenzoic acid)) and reacting the resultant with about equimolar abrin A chain. After the reaction, the Fab'-SS-AA was isolated by removing non-reacted Fab' fragment by chromatography on BLUE SEPHAROSE (commercially available from PHARMACIA) and then removing non-reacted abrin A chain by gel permeation chromatography on ULTROGEL AcA54 (commercially available from IBF).

The Fab'-S-AA according to the present invention was prepared as follows: That is, Fab' was reacted with 13 times by mole of BMME (bismaleimidomethyl ether)/dimethylformamide at 25^{o}C for 1 hour. After removing non-reacted reactants by gel permeation chromatography, the resultant was reacted with equimolar abrin A chain at 25^{o}C for 16 hours to obtain Fab'-S-AA. The isolation of Fab'-S-AA was carried out by exactly the same method as the isolation of Fab'-SS-AA.

### Example 2 Properties of Immunoconjugate

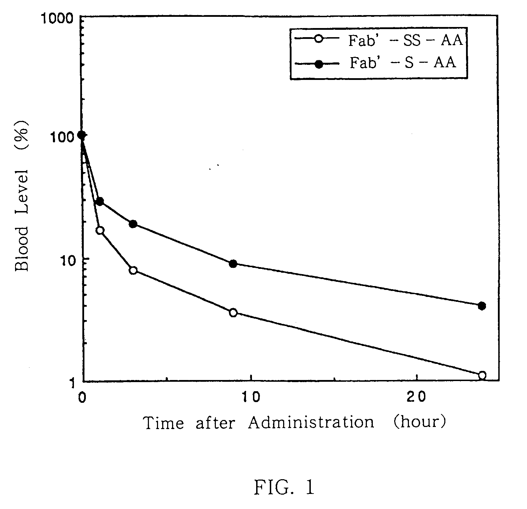

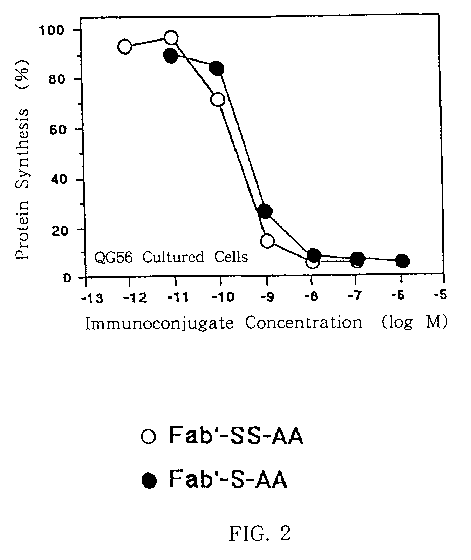

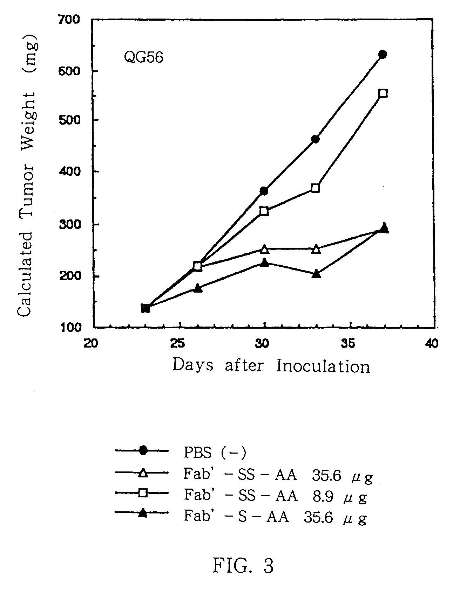

1) Stability of Immunoconjugates in Mouse Blood
   Fab'-S-AA and Fab'-SS-AA were labeled with ¹²⁵I by iodogen method. Each of the labeled conjugates was diluted with 0.1% BSA/PBS(-) and administered to mice (BALB/C, 6 weeks old, male) at a dose of 1 µCi/100µl/mouse. Blood samples were collected after 1, 3, 9 and 24 hours from the administration and sera were prepared from each sample. The radioactivities of TCA-insoluble fractions of the sera were measured and their ratios to the administration dose were calculated.
   As shown in Fig. 1, the retained level of Fab'-S-AA in the blood was higher than that of Fab'-SS-AA, so that the stability of Fab'-S-AA in the blood was higher than that of Fab'-SS-AA. Because of this high retained level in the blood, high effectiveness of the conjugate according to the present invention is expected. Thus, the action of the conjugate was checked.
2) Growth Inhibition of Cancer Cells by Immunoconjugates
   QG-56 cells (human lung squamous cell carcinoma) were cultured under ordinary conditions, and the tumor cells were cultured in wells of a 96-well plate at a concentration of 3 x 10⁴ cells/well. To the wells, various concentrations of the conjugates were added and the plate was incubated overnight. The activities of the conjugates were measured by measuring the inhibition of uptake of ³H-leucine contained in the culture medium into the cells. As a result, as shown in Fig. 2, substantial difference was not observed in the activities of Fab'-S-AA and Fab'-SS-AA. Thus, high effectiveness of Fab'-S-AA as an anti-tumor agent, which has a high stability in the blood is expected.
3) Anti-tumor Activities of Immunotoxin Conjugates in Tumor-bearing Nude Mice
   Small grafts of human lung squamous cell carcinoma (QG-56) cells were inoculated to Balb/c nude mice of 6 weeks old. Fifteen days after the inoculation, two types of immunotoxin conjugates (Fab'-S-AA and Fab'-SS-AA) were administered at a dose of 8.9 or 35.6 µg/mouse four times. The change in the tumor weights and body weights were measured. Thirty one days after the inoculation, the mice were sacrificed and the actual weights of the tumor were measured and outer appearances of the livers were checked. In the mice to which Fab'-S-AA was administered, no abnormal symptoms were observed when compared to those to which another immunotoxin conjugate was administered. As shown in Fig. 3, the change in the tumor weight well corresponded to the growth inhibition of the cancer cells (Fig. 2). Thus, Fab'-S-AA exhibited effective anti-tumor activity.

### Comparative Example

### A. Antibody

Monoclonal antibody AF20 described in Example 1 was used.

### B. Ribosome-inactivating Protein (RIP)

As an RIP, ricin A chain (RA) was used. Ricin A chain was obtained by binding lectin (RCA-60, commercially available from SEIKAGAKU KOGYO CO., LTD.) originated from seeds of Ricinus communis to a lactose-agarose column, equilibrating the column with PBS containing 5% mercaptoethanol (ME-PBS) overnight, and eluting ricin A chain alone with ME-PBS. The fractions containing RA were passed through an asialofetuin column to obtain purified ricin A chain. Analysis by SDS-PAGE and subsequent staining with silver revealed that the obtained ricin A chain was not contaminated with ricin B chain.

### C. Preparation of Immunotoxin Conjugate

A conjugate Fab'-S-RA which is a conjugate of AF20(Fab') and ricin A chain (a conjugate in which Fab' and RA are cross-linked via thioether bond like Fab'-S-AA described in Example 1) was prepared in the same manner as in Example 1. As the cross-linking agent, in addition to BMME, one in which the spacer moiety of the dimaleimide linker is isopropylalcohol was also employed, which gave a little higher yield than BMME.

### D. Anti-tumor Activities of Immunotoxin Conjugates in Tumor-bearing Nude Mice

Small grafts of human lung squamous cell carcinoma cells QG56 were inoculated to Balb/c nude mice of 6 weeks old. Fifteen days after the inoculation, the immunotoxin conjugate (Fab'-S-RA) was administered at a dose of 35.6 µg/mouse four times. The change in the tumor weights and body weights were measured. These were carried out in the same manner as in Example 2.

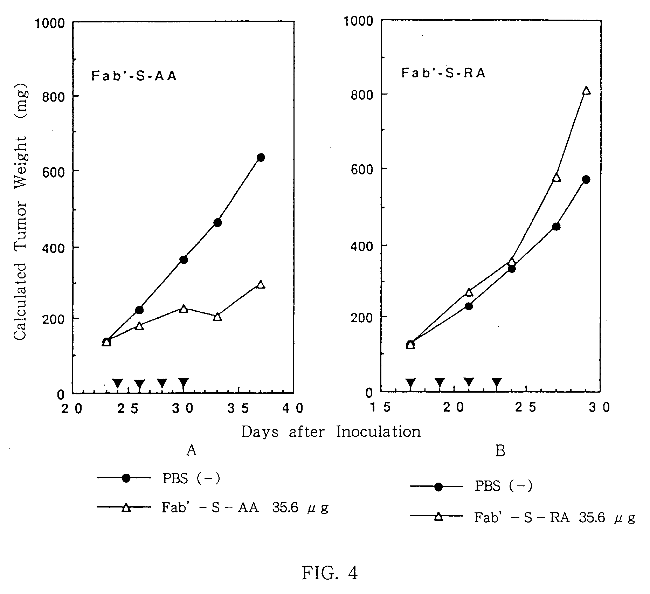

The results are shown in Fig. 4. The results of this Comparative Example are shown in Fig. 4B and the results obtained for Fab'-S-AA shown in Fig. 3 are reproduced in Fig. 4A for comparison. In Fig. 4, 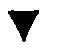 indicates the time points at which the immunotoxin conjugate was administered. As is apparent from the comparison, the immunoconjugate containing abrin A chain had a significantly higher anti-tumor activity than the immunoconjugate having the same structure but containing ricin A chain in place of abrin A chain.

### INDUSTRIAL AVAILABILITY

In the immunoconjugate according to the present invention, abrin A chain is bound to an antibody or an antigen-binding fragment thereof via a cross-linking agent which is not cleaved in the body. Therefore, the immunoconjugate is hardly decomposed in the body and can specifically reach the target cells. Further, in the immunoconjugate according to the present invention, the toxicity of abrin A chain is retained. Therefore, the immunoconjugate according to the present invention is useful as a therapeutic agent for treating tumors and the like.

## Claims

1. An immunoconjugate comprising an antibody or an antigen-binding fragment thereof and abrin A chain, said antibody or antigen-binding fragment thereof and said abrin A chain being bonded to a cross-linking agent via thioether bonds, respectively, which cross-linking agent is not cleaved in the body.

2. The immunoconjugate according to claim 1, which is represented by the formula (I): 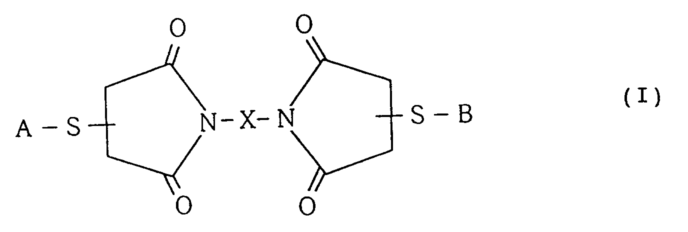 [wherein A represents a residue of said antibody or antigen-binding fragment thereof (A-SH), B represents a residue of abrin A chain (B-SH), and X represents a structure which is not cleaved in the body].

3. The immunoconjugate according to claim 2, wherein X represents CₙH₂ₙ₊₁ (wherein n represents an integer of 1 - 20), CₙH₂ₙ₊₁-O-CₘH₂ₘ₊₁ (wherein n and m independently represent an integer of 1 - 20), -(CH₂CH₂O)ₙ- (wherein n represents an integer of 1 - 200), polyvinyl alcohol (polymerization degree: 1 - 200), polyvinylpyrrolidone (polymerization degree: 1 - 200) or a sugar chain of a polysaccharide (polymerization degree: 1 - 200).

4. The immunoconjugate according to claim 2, wherein X represents -CH₂-O-CH₂- or -CH₂-CH(OH)-CH₂-.

5. The immunoconjugate according to claim 1, wherein said antibody or an antigen-binding fragment thereof is a monoclonal antibody or an antigen-binding fragment thereof, which specifically binds to a tumor cell.

6. The immunoconjugate according to claim 1, wherein said antigen-binding fragment is Fab' fragment.
